# EUROPÄISCHE PATENTSCHRIFT

(11) **EP 0 702 001 B1**
(45) Veröffentlichungstag und Bekanntmachung des Hinweises auf die Patenterteilung: **23.08.2000**
(21) Anmeldenummer: 95113846.0
(22) Anmeldetag: 04.09.1995
(51) Int. Cl.: C07C 279/22, C07C 317/44, A61K 31/155

(54) **Trifluoropropyl-substituierte Benzoylguanidine und ihre Verwendung als Medikament oder Diagnostikum**
Trifluoro-propyl-substituted benzoylguanidinens and their use as medicament or diagnostic agent
Benzoylguanidines trifluoro-propyle substituées et leur utilisation comme médicament ou agent diagnostique

(30) Priorität: 09.09.1994 DE 4432105
(43) Veröffentlichungstag der Anmeldung: 20.03.1996
(73) Patentinhaber: HOECHST AKTIENGESELLSCHAFT, 65929 Frankfurt am Main (DE)
(72) Erfinder: Weichert, Andreas, Dr., D-63329 Egelsbach (DE); Kleemann, Heinz-Werner, Dr., D-65474 Bischofsheim (DE); Lang, Hans-Jochen, Dr., D-65719 Hofheim (DE); Schwark, Jan-Robert, Dr., D-65929 Frankfurt (DE); Albus, Udo, Dr., D-61197 Florstadt (DE); Scholz, Wolfgang, Dr., D-65760 Eschborn (DE)

(56) Entgegenhaltungen:
- EP-A- 0 556 674
- EP-A- 0 602 522
- EP-A- 0 604 852

## Beschreibung

Die Erfindung betrifft Benzoylguanidine l, dadurch gekennzeichnet, daß es sich um
4-(2'-Trifluormethyl-ethyl)-benzoylguanidin-hydrochlorid oder 3-Methylsulfonyl-4-(2'-trifluormethyl-ethyl)-benzoylguanidin-hydrochlorid handelt

Erhalten werden sie aus Verbindungen der Formel II 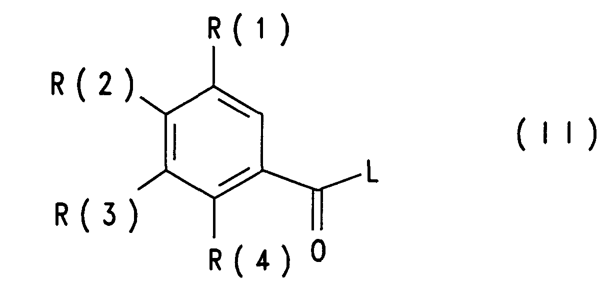 mit der entsprechenden Substitution, in der L eine leicht nucleophil substituierbare Fluchtgruppe bedeutet, durch Umsetzung mit Guanidin.

Die aktivierten Säurederivate der Formel II, worin L eine Alkoxy-, vorzugsweise eine Methoxy-gruppe, eine Phenoxygruppe, Phenylthio-, Methylthio-, 2-Pyridylthiogruppe, einen Stickstoffheterocyclus, vorzugsweise 1-Imidazolyl, bedeutet, erhält man vorteilhaft in an sich bekannter Weise aus den zugrundeliegenden Carbonsäurechloriden (Formel II, L = Cl), die man ihrerseits wiederum in an sich bekannter Weise aus den zugrundeliegenden Carbonsäuren (Formel II, L = OH), beispielsweise mit Thionylchlorid herstellen kann.

Neben den Carbonsäurechloriden der Formel II (L = Cl) lassen sich auch weitere aktivierte Säurederivate der Formel II in an sich bekannter Weise direkt aus den zugrundeliegenden Benzoesäurederivaten (Formel II, L = OH) herstellen, beispielsweise die Methylester der Formel II mit L = OCH₃ durch Behandeln mit gasförmigem HCI in Methanol, die Imidazolide der Formel II durch Behandeln mit Carbonyldiimidazol [L = 1-Imidazolyl, Staab, Angew. Chem. Int. Ed. Engl. 1, 351 -367 (1962)], die gemischten Anhydride II mit Cl-COOC₂H₅ oder Tosylchlorid in Gegenwart von Triethylamin in einem inerten Lösungsmittel, wie auch die Aktivierungen von Benzoesäuren mit Dicyclohexylcarbodiimid (DCC) oder mit O-[(Cyano(ethoxycarbonyl)-methylen)amino]-1,1,3,3-tetramethyluroniumtetrafluorborat ("TOTU") [Proceedings of the 21. European Peptide Symposium, Peptides 1990, Editors E. Giralt and D. Andreu, Escom, Leiden, 1991]. Eine Reihe geeigneter Methoden zur Herstellung von aktivierten Carbonsäurederivaten der Formel II sind unter Angabe von Quellenliteratur in J. March, Advanced Organic Chemistry, Third Edition (John Wiley & Sons, 1985), S. 350 angegeben.

Die Umsetzung eines aktivierten Carbonsäurederivates der Formel II mit Guanidin erfolgt in an sich bekannter Weise in einem protischen oder aprotischen polaren aber inerten organischen Lösungsmittel. Dabei haben sich bei der Umsetzung der Benzoesäuremethylester (II, L = OMe) mit Guanidin Methanol, Isopropanol oder THF von 20°C bis zur Siedetemperatur dieser Lösungsmittel bewährt. Bei den meisten Umsetzungen von Verbindungen II mit salzfreiem Guanidin wurde vorteilhaft in aprotischen inerten Lösungsmitteln wie THF, Dimethoxyethan, Dioxan gearbeitet. Aber auch Wasser kann unter Gebrauch einer Base wie beispielsweise NaOH als Lösungsmittel bei der Umsetzung von II mit Guanidin verwendet werden.

Wenn L = Cl bedeutet, arbeitet man vorteilhaft unter Zusatz eines Säurefängers, z. B. in Form von überschüssigem Guanidin zur Abbindung der Halogenwasserstoffsäure.

Ein Teil der zugrundeliegenden Benzoesäurederivate der Formel II ist bekannt und in der Literatur beschrieben. Die unbekannten Verbindungen der Formel II können nach literaturbekannten Methoden hergestellt werden. Die erhaltenen Benzoesäuren werden nach einer der oben beschriebenen Verfahrensvarianten zu erfindungsgemäßen Verbindungen I umgesetzt.

Die Einführung einiger Substituenten in 2-, 3-, 4- und 5-Stellung gelingt durch literaturbekannte Methoden des Palladium-vermittelten cross-couplings von Arylhalogeniden bzw. Aryltriflaten mit beispielsweise Organostannanen, Organoboronsäuren oder Organoboranen oder Organokupfer- bzw. Organozinkverbindungen.

Die Verbindungen I sind substituierte Acylguanidine.

Prominentester Vertreter der Acylguanidine ist das Pyrazinderivat Amilorid, das als kaliumsparendes Diuretikum in der Therapie Verwendung findet. Zahlreiche weitere Verbindungen vom Amilorid-Typ werden in der Literatur beschrieben, wie beispielsweise Dimethylamilorid oder Ethylisopropylamilorid. 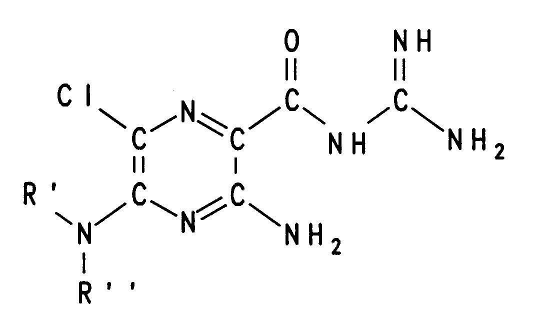
Amilorid: R`,R`` = H
Dimethylamilorid: R`,R`` = CH₃
Ethylisopropylamilorid: R` = C₂H₅, R`` = CH(CH₃)₂

Darüberhinaus sind Untersuchungen bekannt geworden, die auf antiarrhythmische Eigenschaften von Amilorid hinweisen (Circulation 79, 1257 - 63 (1989)). Einer breiten Anwendung als Antiarrhythmikum steht jedoch entgegen, daß dieser Effekt nur schwach ausgeprägt ist und von einer blutdrucksenkenden und saluretischen Wirkung begleitet auftritt und diese Nebenwirkungen bei der Behandlung von Herz-Rhythmusstörungen unerwünscht sind.

Hinweise auf antiarrhythmische Eigenschaften des Amilorids wurden auch bei Experimenten an isolierten Tierherzen erhalten (Eur. Heart J. 9 (suppl.1): 167 (1988) (book of abstracts)). So wurde beispielsweise an Rattenherzen gefunden, daß ein künstlich ausgelöstes Kammerflimmern durch Amilorid völlig unterdrückt werden konnte. Noch potenter als Amilorid war in diesem Modell das oben erwähnte Amiloridderivat Ethylisopropylamilorid.

In der US-Patentschrift 5 091 394 und in der europäischen Offenlegungsschrift 0 556 674 A 1 (HOE 92/F 034) werden Benzoylguanidine beschrieben, die jedoch keine (teil-)fluorierten Alkyl- bzw. Alkenyl-Seitenketten zur metabolischen Stabilisierung aufweisen.

Im US-Patent 3 780 027 werden Acylguanidine beansprucht, die strukturell den Verbindungen I ähnlich sind und sich von im Handel befindlichen Schleifendiuretika, wie Bumetanid, ableiten. Entsprechend wird für diese Verbindungen eine starke salidiuretische Wirksamkeit berichtet.

Es war daher überraschend, daß die erfindungsgemäßen Verbindungen keine unerwünschten und nachteiligen salidiuretischen, jedoch sehr gute antiarrhythmische Eigenschaften aufweisen, wie sie beispielsweise bei Sauerstoffmangelerscheinungen auftreten. Die Verbindungen sind infolge ihrer pharmakologischen Eigenschaften als antiarrhythmische Arzneimittel mit cardioprotektiver Komponente zur Infarktprophylaxe und der Infarktbehandlung sowie zur Behandlung der angina pectoris hervorragend geeignet, wobei sie auch präventiv die pathophysiologischen Vorgänge beim Entstehen ischämisch induzierter Schäden, insbesondere bei der Auslösung ischämisch induzierter Herzarrhythmien, inhibieren oder stark vermindern. Wegen ihrer schützenden Wirkungen gegen pathologische hypoxische und ischämische Situationen können die erfindungsgemäßen Verbindungen I infolge Inhibition des zellulären Na⁺/H⁺-Austauschmechanismus als Arzneimittel zur Behandlung aller akuten oder chronischen durch Ischämie ausgelösten Schäden oder dadurch primär oder sekundär induzierten Krankheiten verwendet werden. Dies betrifft ihre Verwendung als Arzneimittel für operative Eingriffe, z.B. bei OrganTransplantationen, wobei die Verbindungen sowohl für den Schutz der Organe im Spender vor und während der Entnahme, zum Schutz entnommener Organe beispielsweise bei Behandlung mit oder deren Lagerung in physiologischen Badflüssigkeiten, wie auch bei der Überführung in den Empfängerorganismus verwendet werden können. Die Verbindungen sind ebenfalls wertvolle, protektiv wirkende Arzneimittel bei der Durchführung angioplastischer operativer Eingriffe beispielsweise am Herzen wie auch an peripheren Gefäßen. Entsprechend ihrer protektiven Wirkung gegen ischämisch induzierte Schäden sind die Verbindungen auch als Arzneimittel zur Behandlung von Ischämien des Nervensystems, insbesondere des ZNS, geeignet, wobei sie z.B. zur Behandlung des Schlaganfalls oder des Hirnödems geeignet sind. Darüberhinaus eignen sich die erfindungsgemäßen Verbindungen I ebenfalls zur Behandlungen von Formen des Schocks, wie beispielweise des allergischen, cardiogenen, hypovolämischen und des bakteriellen Schocks.

Darüberhinaus zeichnen sich die erfindungsgemäßen Verbindungen I durch starke inhibierende Wirkung auf die Proliferationen von Zellen, beispielsweise der Fibroblasten- Zellproliferation und der Proliferation der glatten Gefäßmuskelzellen, aus. Deshalb kommen die Verbindungen I als wertvolle Therapeutika für Krankheiten infrage, bei denen die Zellproliferation eine primäre oder sekundäre Ursache darstellt, und können deshalb als Antiatherosklerotika, Mittel gegen diabetische Spätkomplikationen, Krebserkrankungen, fibrotische Erkrankungen wie Lungenfibrose, Leberfibrose oder Nierenfibrose, Organhypertrophien und - hyperplasien, insbesondere bei Prostatahyperplasie bzw. Prostatahypertrophie verwendet werden.

Die erfindungsgemäßen Verbindungen sind wirkungsvolle Inhibitoren des zellulären Natrium-Protonen-Antiporters (Na⁺/H⁺-Exchanger), der bei zahlreichen Erkrankungen (Essentielle Hypertonie, Atherosklerose, Diabetes usw.) auch in solchen Zellen erhöht ist, die Messungen leicht zugänglich sind, wie beispielsweise in Erythrocyten, Thrombocyten oder Leukozyten. Die erfindungsgemäßen Verbindungen eignen sich deshalb als hervorragende und einfache wissenschaftliche Werkzeuge, beispielsweise in ihrer Verwendung als Diagnostika zur Bestimmung und Unterscheidung bestimmter Formen der Hypertonie, aber auch der Atherosklerose, des Diabetes, proliferativer Erkrankungen usw.. Darüber hinaus sind die Verbindungen I für die präventive Therapie zur Verhinderung der Genese des Bluthochdrucks, beispielweise der essentiellen Hypertonie, geeignet.

Arzneimittel, die eine Verbindung I enthalten, können dabei oral, parenteral, intravenös, rektal oder durch Inhalation appliziert werden, wobei die bevorzugte Applikation von dem jeweiligen Erscheinungsbild der Erkrankung abhängig ist. Die Verbindungen I können dabei allein oder zusammen mit galenischen Hilfsstoffen zur Anwendung kommen, und zwar sowohl in der Veterinär- als auch in der Humanmedizin.

Welche Hilfsstoffe für die gewünschte Arzneimittelformulierung geeignet sind, ist dem Fachmann auf Grund seines Fachwissens geläufig. Neben Lösemitteln, Gelbildnern, Suppositorien-Grundlagen, Tablettenhilfsstoffen, und anderen Wirkstoffträgern können beispielsweise Antioxidantien, Dispergiermittel, Emulgatoren, Entschäumer, Geschmackskorrigentien, Konservierungsmittel, Lösungsvermittler oder Farbstoffe verwendet werden.

Für eine orale Anwendungsform werden die aktiven Verbindungen mit den dafür geeigneten Zusatzstoffen, wie Trägerstoffen, Stabilisatoren oder inerten Verdünnungsmittel vermischt und durch die üblichen Methoden in die geeigneten Darreichungsformen gebracht, wie Tabletten, Dragees, Steckkapseln, wäßrige, alkoholische oder ölige Lösungen. Als inerte Träger können z. B. Gummi arabicum, Magnesia, Magnesiumcarbonat, Kaliumphosphat, Milchzucker, Glucose oder Stärke, insbesondere Maisstärke, verwendet werden. Dabei kann die Zubereitung sowohl als Trocken- als auch als Feuchtgranulat erfolgen. Als ölige Trägerstoffe oder als Lösemittel kommen beispielsweise pflanzliche oder tierische Öle in Betracht, wie Sonnenblumenöl oder Lebertran.

Zur subkutanen oder intravenösen Applikation werden die aktiven Verbindungen, gewünschtenfalls mit den dafür üblichen Substanzen wie Lösungsvermittler, Emulgatoren oder weiteren Hilfsstoffen in Lösung, Suspension oder Emulsion gebracht. Als Lösungsmittel kommen z. B. in Frage: Wasser, physiologische Kochsalzlösung oder Alkohole, z. B. Ethanol, Propanol, Glycerin, daneben auch Zuckerlösungen wie Glucose- oder Mannitlösungen, oder auch eine Mischung aus den verschiedenen genannten Lösungsmitteln.

Als pharmazeutische Formulierung für die Verabreichung in Form von Aerosolen oder Sprays sind geeignet z. B. Lösungen, Suspensionen oder Emulsionen des Wirkstoffes I in einem pharmazeutisch unbedenklichen Lösungsmittel, wie insbesondere Ethanol oder Wasser, oder einem Gemisch solcher Lösungsmittel.

Die Formulierung kann nach Bedarf auch noch andere pharmazeutische Hilfsstoffe wie Tenside, Emulgatoren und Stabilisatoren sowie ein Treibgas enthalten. Eine solche Zubereitung enthält den Wirkstoff üblicherweise in einer Konzentration von etwa 0,1 bis 10, insbesondere von etwa 0,3 bis 3 Gew.-%.

Die Dosierung des zu verabreichenden Wirkstoffs I und die Häufigkeit der Verabreichung hängen von der Wirkstärke und Wirkdauer der verwendeten Verbindungen ab; außerdem auch von Art und Stärke der zu behandelnden Krankheit sowie von Geschlecht, Alter, Gewicht und individueller Ansprechbarkeit des zu behandelnden Säugers.
Im Durchschnitt beträgt die tägliche Dosis einer Verbindung I bei einem etwa 75 kg schweren Patienten mindestens 0,001 mg/kg, vorzugsweise 0,01 mg/kg, bis höchstens 10 mg/kg, vorzugsweise 1 mg/kg Körpergewicht. Bei akuten Ausbrüchen der Krankheit, etwa unmittelbar nach Erleiden eines Herzinfarkts, können auch noch höhere und vor allem häufigere Dosierungen notwendig sein, z. B. bis zu 4 Einzeldosen pro Tag. Insbesondere bei i.v. Anwendung, etwa bei einem Infarktpatienten auf der Intensivstation können bis zu 200 mg pro Tag notwendig werden.

Liste der Abkürzungen:

| | |
|---|---|
| MeOH | Methanol |
| DMF | N,N-Dimethylformamid |
| RT | Raumtemperatur |
| EE | Ethylacetat (EtOAc) |
| Smp | Schmelzpunkt |
| THF | Tetrahydrofuran |
| eq. | Äquivalent |

### Experimenteller Teil

Allgemeine Vorschrift zur Herstellung von Benzoyl-guanidinen (I)
Variante A: aus Benzoesäuren (II, L = OH)

1.0 eq. des Benzoesäurederivates der Formel II löst bzw. suspendiert man in wasserfreiem THF (5 ml/mmol) und versetzt sodann mit 1.1 eq. Carbonyldiimidazol. Nach dem Rühren über 2 Stunden bei RT werden 5.0 eq. Guanidin in die Reaktionslösung eingetragen. Nach dem Rühren über Nacht destilliert man das THF unter vermindertem Druck (Rotationsverdampfer) ab, versetzt mit Wasser, stellt mit 2N HCI auf pH 6 bis 7 und filtriert das entsprechende Benzoylguanidin (Formel I) ab. Die so erhaltenen Benzoylguanidine können durch Behandeln mit wäßriger, methanolischer oder etherischer Salzsäure oder anderen pharmakologisch verträglichen Säuren in die entsprechenden Salze übergeführt werden.

Allgemeine Vorschrift zur Herstellung von Benzoyl-guanidinen (I)
Variante B: aus Benzoesäure-alkylestern (II, L = O-Alkyl)

1.0 eq. des Benzoesäure-alkylesters der Formel II sowie 5.0 eq. Guanidin (freie Base) werden in Isopropanol gelöst oder in THF suspendiert und bis zum vollständigen Umsatz (Dünnschichtkontrolle) zum Rückfluß erhitzt (typische Reaktionszeit 2 bis 5 h). Das Lösungsmittel wird unter vermindertem Druck (Rotationsverdampfer) abdestilliert, in EE aufgenommen und 3 x mit NaHCO₃-Lösung gewaschen. Es wird über Na₂SO₄ getrocknet, das Lösungsmittel im Vakuum abdestilliert und an Kieselgel mit einem geeigneten Laufmittel, z. B. EE/MeOH 5 : 1 chromatographiert.
(Salzbildung vergleiche Variante A)

### Beispiel 1:

4-(2'-Trifluormethyl-ethyl)-benzoylguanidin-hydrochlorid:
Farblose Kristalle, Smp. 168 - 72°C

### Syntheseweg:

a) 4-(2'-Trifluormethyl-ethenyl)-benzoesäuremethylester durch Kupplung des mit Zinkchlorid transmetallierten Grignardreagenzes von 2-Brom-2-trifluormethylethen (3 Äquivalente) mit 4-Brom-benzoesäuremethylester in THF unter Rückfluß, in Gegenwart von 0.6 Äquivalenten Palladiumacetat und 0.1 Äquivalenten Triphenylphosphin und 0.015 Äquivalenten Kupfer(I)iodid. Wäßrige Aufarbeitung und Chromatographie mit n-Heptan/Essigester 4 : 1 liefert ein gelbes Öl.
b) 4-(2'-Trifluormethyl-ethyl)-benzoesäuremethylester aus 2 a) mittels Hydrierung in Gegenwart von Palladium auf Aktivkohle in Methanol innerhalb 2 h. Nach Entfernen des Lösungsmittels wird ein farbloses Öl erhalten.
c) 4-(2'-Trifluormethyl-ethyl)-benzoylguanidin-hydrochlorid aus 2 b) nach Variante B.

### Beispiel 2

3-Methylsulfonyl-4-(2'-trifluormethyl-ethyl)-benzoylguanidin-hydrochlorid: Farblose Kristalle, Smp. 197°C
Syntheseweg:
a) 3-Methylsulfonyl-4-(2'-trifluormethyl-ethenyl)-benzosäuremethylester analog 2 a) unter Verwendung von 4-Brom-3-methylsulfonyl-benzoesäuremethylester als Kupplungspartner.
   bräunliches Wachs nach Chromatographie mit n-Heptan/Essigester 3 : 2.
b) 3-Methylsulfonyl-4-(2'-trifluormethyl-ethyl)-benzoesäuremethylester analog 3 b), farblose Kristalle, Smp. 128°C.
c) 3-Methylsulfonyl-4-(2'-trifluormethyl-ethyl)-benzoylguanidin-hydrochlorid nach Guanidierungsvariante B.

## Patentansprüche

1. Benzoylguanidine, dadurch gekennzeichnet, daß es sich um 4-(2'-Trifluormethyl-ethyl)-benzoylguanidin-hydrochlorid oder 3-Methylsulfonyl-4-(2'-trifluormethyl-ethyl)-benzoylguanidin-hydrochlorid handelt

2. Verwendung eines Benzoylguanidins nach Anspruch 1 zur Herstellung eines Medikaments zu Behandlung von Arrhythmien.

3. Verwendung eines Benzoylguanidins nach Anspruch 1 zur Herstellung eines Medikaments zur Behandlung oder Prophylaxe des Herzinfarkts.

4. Verwendung eines Benzoylguanidins nach Anspruch 1 zur Herstellung eines Medikaments zur Behandlung oder Prophylaxe der Angina Pectoris.

5. Verwendung eines Benzoylguanidins nach Anspruch 1 zur Herstellung eines Medikaments zur Behandlung oder Prophylaxe von ischämischen Zuständen des Herzens.

6. Verwendung eines Benzoylguanidins nach Anspruch 1 zur Herstellung eines Medikaments zur Behandlung oder Prophylaxe von ischämischen Zuständen des peripheren und zentralen Nervensystems und des Schlaganfalls.

7. Verwendung eines Benzoylguanidins nach Anspruch 1 zur Herstellung eines Medikaments zur Behandlung oder Prophylaxe von ischämischen Zuständen peripherer Organe und Gliedmaßen.

8. Verwendung eines Benzoylguanidins nach Anspruch 1 zur Herstellung eines Medikaments zur Behandlung von Schockzuständen.

9. Verwendung eines Benzoylguanidins nach Anspruch 1 zur Herstellung eines Medikaments zum Einsatz bei chirurgischen Operationen und Organtransplantationen.

10. Verwendung eines Benzoylguanidins nach Anspruch 1 zur Herstellung eines Medikaments zur Konservierung und Lagerung von Transplantaten für chirurgische Maßnahmen.

11. Verwendung eines Benzoylguanidins nach Anspruch I zur Herstellung eines Medikaments zur Behandlung von Krankheiten, bei denen die Zellproliferation eine primäre oder sekundäre Ursache darstellt.

12. Heilmittel, enthaltend eine wirksame Menge eines Benzoylguanidins nach Anspruch 1.

## Claims

1. A benzoylguanidine which is 4-(2'-trifluoromethylethyl)benzoylguanidine hydrochloride or 3-methylsulfonyl-4-(2'-trifluoromethylethyl)benzoylguanidine hydrochloride.

2. Use of a benzoylguanidine as claimed in claim 1 for preparing a medicament for the treatment of arrhythmias.

3. Use of a benzoylguanidine as claimed in claim 1 for preparing a medicament for the treatment or prophylaxis of cardiac infarction.

4. Use of a benzoylguanidine as claimed in claim 1 for preparing a medicament for the treatment or prophylaxis of angina pectoris.

5. Use of a benzoylguanidine as claimed in claim 1 for preparing a medicament for the treatment or prophylaxis of ischemic conditions of the heart.

6. Use of a benzoylguanidine as claimed in claim 1 for preparing a medicament for the treatment or prophylaxis of ischemic conditions of the peripheral and central nervous system and of stroke.

7. Use of a benzoylguanidine as claimed in claim 1 for preparing a medicament for the treatment or prophylaxis of ischemic conditions of peripheral organs and limbs.

8. Use of a benzoylguanidine as claimed in claim 1 for preparing a medicament for the treatment of shock conditions.

9. Use of a benzoylguanidine as claimed in claim 1 for preparing a medicament for employment in surgical operations and organ transplantations.

10. Use of a benzoylguanidine as claimed in claim 1 for preparing a medicament for the preservation and storage of transplants for surgical procedures.

11. Use of a benzoylguanidine as claimed in claim 1 for preparing a medicament for the treatment of diseases in which cell proliferation represents a primary or secondary cause.

12. A medicine, containing an effective quantity of a benzoylguanidine as claimed in claim 1.

## Revendications

1. Benzoylguanidines, caractérisées en ce qu'il s'agit du chlorhydrate de 4-(2'-trifluorométhyl-éthyl) benzoylguanidine ou du chlorhydrate de 3-méthylsulfonyl-4-(2'-trifluorométhyl-éthyl)-benzoylguanidine.

2. Utilisation d'une benzoylguanidine selon la revendication 1, pour la fabrication d'un médicament destiné au traitement d'arythmies.

3. Utilisation d'une benzoylguanidine selon la revendication 1, pour la fabrication d'un médicament destiné au traitement ou à la prophylaxie de l'infarctus du myocarde.

4. Utilisation d'une benzoylguanidine selon la revendication 1, pour la fabrication d'un médicament destiné au traitement ou à la prophylaxie de l'angine de poitrine.

5. Utilisation d'une benzoylguanidine selon la revendication 1, pour la fabrication d'un médicament destiné au traitement ou à la prophylaxie d'états ischémiques du coeur.

6. Utilisation d'une benzoylguanidine selon la revendication 1, pour la fabrication d'un médicament destiné au traitement ou à la prophylaxie d'états ischémiques du système nerveux périphérique et central et de l'apoplexie cérébrale.

7. Utilisation d'une benzoylguanidine selon la revendication 1, pour la fabrication d'-un médicament destiné au traitement ou à la prophylaxie d'états ischémiques d'organes périphériques et des membres.

8. Utilisation d'une benzoylguanidine selon la revendication 1, pour la fabrication d'un médicament destiné au traitement d'états de choc.

9. Utilisation d'une benzoylguanidine selon la revendication 1, pour la fabrication d'un médicament destiné à l'utilisation dans des opérations chirurgicales et des greffes d'organes.

10. Utilisation d'une benzoylguanidine selon la revendication 1, pour la fabrication d'un médicament destiné à la conservation et au stockage de transplants pour des opérations chirurgicales.

11. Utilisation d'une benzoylguanidine selon la revendication 1, pour la fabrication d'un médicament destiné au traitement de maladies dans lesquelles la prolifération cellulaire représente une cause primaire ou secondaire.

12. Médicament, contenant une quantité efficace d'une benzoylguanidine selon la revendication 1.
